# EUROPEAN PATENT SPECIFICATION

(11) **EP 3 102 565 B1**
(45) Date of publication and mention of the grant of the patent: **02.01.2019**
(21) Application number: 15708328.8
(22) Date of filing: 03.02.2015
(51) Int. Cl.: C07D 239/557, C07D 413/12

(54) **PROCESSES FOR THE PREPARATION OF INTERMEDIATES OF RALTEGRAVIR**
VERFAHREN ZUR HERSTELLUNG VON RALTEGRAVIR-INTERMEDIATEN
PROCÉDÉS DE PRÉPARATION D' INTERMÉDIAIRES DE RALTÉGRAVIR

(30) Priority: 03.02.2014 IN 471CH2014
(43) Date of publication of application: 14.12.2016
(73) Proprietor: Mylan Laboratories Ltd., Hyderabad 500033 (IN)
(72) Inventor: VELLANKI, Siva Ram Prasad, Hyderabad 500033 (IN); BALUSU, Raja Babu, Hyderabad 500033 (IN); MITCHANAGATLA, Kiran, Hyderabad 500033 (IN)
(74) Representative: FRKelly
(86) International application number: PCT/IB2015/050808
(87) International publication number: WO 2015/114608

(56) References cited:
- WO-A1-2012/103105
- US-B2- 7 754 731
- GUY R HUMPHREY ET AL: "Development of a Second-Generation, Highly Efficient Manufacturing Route for the HIV Integrase Inhibitor Raltegravir Potassium", ORGANIC PROCESS RESEARCH AND DEVELOPMENT, AMERICAN CHEMICAL SOCIETY, US, vol. 15, no. 1, 21 January 2011 (2011-01-21), pages 73-83, XP009159108, ISSN: 1083-6160, DOI: 10.1021/OP100257R [retrieved on 2010-11-16]
- Peter Wuts ET AL: "Protection for the Amino Group" In: "Greene's Protective Groups in Organic Synthesis", 10 April 2006 (2006-04-10), John Wiley & Sons, Inc, Hoboken, NJ, USA, XP055165145, ISBN: 978-0-47-169754-1 pages 696-926, DOI: 10.1002/9780470053485.ch7, entries 21-22; page 752

## Description

### BACKGROUND OF THE INVENTION

### FIELD OF THE INVENTION

The present disclosure relates generally to methods of synthesis of pharmaceutical products, and more specifically to a process for the preparation of 2-(2-amino propan-2-yl)-N-(4-fluorobenzyl)-5-hydroxy-1-methyl-6-oxo-1,6-dihydro-pyrimidine-4-carboxamide and its further conversion into N-(4-fluorobenzyl)-5-hydroxy-1-methyl-2-(2-{[(5-methyl-1,3,4-oxadiazol-2-yl)carbonyl]amino}-2-propanyl)-6-oxo-1,6-dihydro-4-pyrimidinecarboxamide and pharmaceutically acceptable salts thereof.

### BACKGROUND OF THE INVENTION

Raltegravir is an antiretroviral drug, which in its potassium salt form, is marketed under the brand name ISENTRESS® by Merck & Co. It is often used in combination with other anti-retroviral drugs to treat human immunodeficiency virus (HIV) infection. Raltegravir is a first line HIV-integrase strand transfer inhibitor drug that targets integrase, an HIV enzyme that integrates viral genetic material into human chromosomes. Raltegravir potassium is chemically known as 4-[N-(4-fluorobenzyl) carbamoyl}-1-methyl-2-{1-methyl-1-(5-methyl-1,3,4-oxadiazol-2-ylcarboxamido)ethyl}-6-oxo-1,6-dihydropyrimidin-5-olate potassium salt. It has a structure represented below by Formula I.

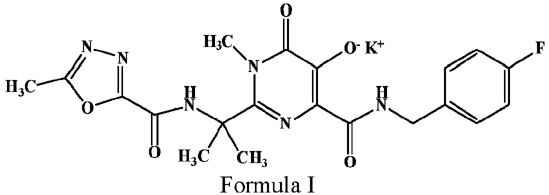

Raltegravir and its pharmaceutically acceptable salts are disclosed in U.S. Patent No. 7,169,780, which is hereby incorporated by reference.

The process for the preparation of Raltegravir is disclosed in U.S. Patent No. 7,169,780 and U.S. Patent App. Pub. No. 2010/0280244. Guy et al Organic Process and Development 15 (1), 2011, pages 73-83 discloses a similar process and Example 8 of WO2012/103105 discloses another process for the preparation of Raltegravir.

There exists a need to provide a cost-effective and simple process for synthesizing raltegravir. The present invention provides an improved and simple process which employs debenzylation of a benzyl-protected intermediate with hydrobromic acid to produce raltegravir.

### SUMMARY OF THE INVENTION

One aspect of the present disclosure is to provide a process for preparation of 2-(2-amino propan-2-yl)-N-(4-fluorobenzyl)-5-hydroxy-1-methyl-6-oxo-1,6-dihydro pyrimidine-4-carboxamide (shown below as Formula III) by debenzylation of benzyl(2-{4-[(4-fluorobenzyl)carbamoyl]-5-hydroxy-1-methyl-6-oxo-1,6-dihydropyrimidin-2-yl} propan-2-yl)carbamate (shown below as Formula IV). Formula III may be formed as an intermediate during the synthesis of raltegravir.

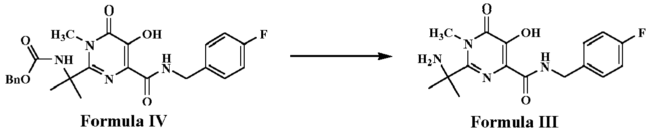

### DETAILED DESCRIPTION OF THE INVENTION

It is to be understood that the description of the present invention has been simplified to illustrate elements that are relevant for a clear understanding of the invention, while eliminating, for purposes of clarity, other elements that may be well known.

The present invention discloses novel methods for producing intermediates in the synthesis of raltegravir.

One aspect of the present disclosure is to provide a process for preparation of 2-(2-amino propan-2-yl)-N-(4-fluorobenzyl)-5-hydroxy-1-methyl-6-oxo-1,6-dihydro pyrimidine-4-carboxamide (shown below, and hence forward referred to as as 'Formula III') by debenzylation of benzyl(2-{4-[(4-fluorobenzyl)carbamoyl]-5-hydroxy-1-methyl-6-oxo-1,6-dihydropyrimidin-2-yl}propan-2-yl)carbamate (shown below, and hence forward referred to as 'Formula IV'). Formula III may be formed as an intermediate during the synthesis of raltegravir.

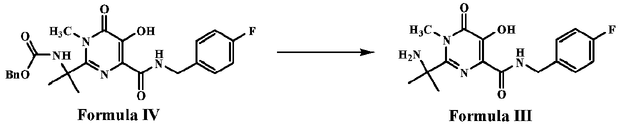

In one embodiment of the present invention, the process for the preparation of a compound of Formula III is carried out by the following steps:
a) debenzylation of a compound of Formula IV in the presence of an acid; and
b) isolating a compound of Formula III, wherein the acid is hydrobromic acid and wherein debenzylation of the compound of formula IV is carried out at a temperature between 40 °C and 100 °C.

According to the present disclosure, Formula IV may be debenzylated to produce Formula III. This reaction may occur in the presence of an acid, which may be organic or inorganic. Within the context of the present disclosure, suitable organic acids include, as examples, acetic acid, trifluoroacetic acid, trifluoromethanesulfuric acid, and formic acid. Suitable inorganic acids include, as examples, hydrochloric acid, hydrobromic acid, sulfuric acid, hydrofluoric acid, boric acid, tetrafluoroboric acid, and orthophosphoric acid. In some embodiments of the disclosure, it has been found that the aqueous form of the inorganic acid is particularly useful for carrying out this step. In particular, aqueous hydrobromic acid was found to be effective.

According to the present disclosure, the debenzylation of Formula IV to give Formula III may be carried out in the presence of a solvent. Within the context of the present invention, suitable solvents include, as examples, acetone, methanol, ethanol, n-propanol, 2-propanol, and mixtures thereof.

According to the present invention, the debenzylation of Formula IV is carried out at a temperature ranging from about 40°C to about 100 °C. It has been found that a temperature range of about 60 °C to about 65 °C is particularly effective for carrying out the debenzylation reaction.

According to the present disclosure, after completion of the debenzylation reaction, the reaction mass may then be cooled to about 5 °C to about 20 °C, at which time the pH of the solution may be adjusted to about 7.0 to 12.0, particularly 7.0 to 8.0. The mixture may then be cooled further to about 0 - 5 °C and stirred to isolate Formula III. In some embodiments of the invention, cooling to a temperature between about 10 and 15 °C prior to adjusting the pH has been found to be particularly effective when carrying out this reaction.. After isolation, the product may then be optionally washed with a solvent to enhance purification. Examples of suitable washing solvents include alcohol solvents, as examples, methanol, ethanol, n-propanol and 2-propanol.

In another aspect of the present invention, the obtained Formula III may be further converted into raltegravir or pharmaceutically acceptable salts thereof by processes disclosed in U.S. Patent No. 7,754,731, PCT Publication Nos. WO2011024192 and WO 2010140156, as well as Indian Patent Application Serial No. 736/CHE/2012.

With all of the reactions disclosed above, one of skill in the art will recognize that the reaction conditions (e.g., reaction time or temperature) may be adjusted to achieve appropriate yield without undertaking undue experimentation and without departing from the scope of the present disclosure.

Raltegravir or pharmaceutically acceptable salts thereof, prepared by the processes disclosed in the present invention may be incorporated into a pharmaceutical formulation for the treatment of HIV in human patients. Numerous types of pharmaceutical formulations may be employed, including tablets, chewable tablets, and oral suspensions. When formulated as a tablet, the formulation may include such excipients as calcium phosphate dibasic anhydrous, hypromellose 2208, lactose monohydrate, magnesium stearate, microcrystalline cellulose, poloxamer 407 (contains 0.01% butylated hydroxytoluene as antioxidant), sodium stearyl fumarate. In addition, the tablet may include a film coating that may contain the following inactive ingredients: black iron oxide, polyethylene glycol 3350, polyvinyl alcohol, red iron oxide, talc and titanium dioxide. In some embodiments, the raltegravir or pharmaceutically acceptable salts thereof may be included in a chewable tablet. Such formulations may include, as examples of appropriate excipients, ammonium hydroxide, crospovidone, ethylcellulose 20 cP, fructose, hydroxypropyl cellulose, hypromellose 2910/6cP, magnesium stearate, mannitol, medium chain triglycerides, monoammonium glycyrrhizinate, natural and artificial flavors (orange, banana, and masking that contains aspartame), oleic acid, PEG 400, red iron oxide, saccharin sodium, sodium citrate dihydrate, sodium stearyl fumarate, sorbitol, sucralose and yellow iron oxide. In other embodiments, the pharmaceutical formulation may be an oral suspension. The formulation intended for oral suspension may include excipients such as ammonium hydroxide, artificial flavorings, natural flavorings, carboxymethylcellulose sodium, crospovidone, ethylcellulose 20 cP, fructose, hydroxypropyl cellulose, hypromellose 2910/6 cP, macrogol/PEG 400, magnesium stearate, maltodextrin, mannitol, medium chain triglycerides, microcrystalline cellulose, monoammonium glycyrrhizinate, oleic acid, sorbitol, sucralose, and sucrose.

In treatment of patients with HIV, raltegravir or pharmaceutically acceptable salts thereof, prepared by the processes disclosed in the present invention may also be administered in conjunction with other active pharmaceutical ingredients, including efavirenz, fosamprenavir, ritonavir, tipranavir, rifampin, tenofovir, lamivudine, and emtricitabine.

In view of the above description and the examples below, one of ordinary skill in the art will be able to practice the invention as claimed without undue experimentation. The foregoing will be better understood with reference to the following examples that detail certain procedures for the preparation of molecules, compositions and formulations according to the present invention. All references made to these examples are for the purposes of illustration. The following examples should not be considered exhaustive, but merely illustrative of only a few of the many aspects and embodiments contemplated by the present disclosure.

### Example 1: Preparation of Formula III

A mass of 730 g of aqueous hydrobromic acid was added to 100 g of benzyl (2-{4-[(4-fluorobenzyl) carbamoyl]-5-hydroxy-1-methyl-6-oxo-1,6-dihydropyrimidin-2-yl}propan-2-yl)-carbamate at 28 ± 3 °C and stirred for 30 minutes. The reaction mixture temperature was raised to 62 ± 3 °C and stirring continued for 2 hours. After completion of the reaction, the reaction mixture was cooled to 28 ± 3 °C and 300 mL of purified water was added. The reaction mixture was stirred and cooled to 12 ± 3 °C. The pH of the reaction mixture was adjusted to 7.0 - 8.0 with a sodium hydroxide solution and further cooled to 3 ± 2 °C. The reaction mixture was stirred for about 3-4 hrs. The product was filtered then washed with water. Methanol (500 mL) was added to the wet material at 28 ±3 °C and stirred for 1 hour to obtain a solid. The solid was filtered then washed with methanol. The product was dried under vacuum at 55 ± 3 °C to get 2-(2-amino propan-2-yl)-N-(4-fluorobenzyl)-5-hydroxy-1-methyl-6-oxo-1,6-dihydro pyrimidine-4-carboxamide (Formula III).

## Claims

1. A process for the preparation of 2-(2-amino propan-2-yl)-N-(4-fluorobenzyl)-5-hydroxy-1-methyl-6-oxo-1,6-dihydro pyrimidine-4-carboxamide of formula III, comprising:
a. debenzylation of benzyl(2-{4-[(4-fluorobenzyl)carbamoyl]-5-hydroxy-1-methyl-6-oxo-1,6-dihydropyrimidin-2-yl}propan-2-yl)carbamate (Formula IV) in the presence of an acid; and
b. isolating 2-(2-amino propan-2-yl)-N-(4-fluorobenzyl)-5-hydroxy-1-methyl-6-oxo-1,6-dihydro pyrimidine-4-carboxamide (Formula III). 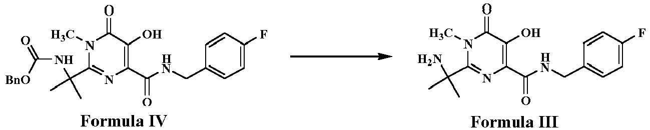 wherein the acid is hydrobromic acid; and wherein debenzylation of compound of Formula IV is carried out at a temperature between 40 °C and 100 °C.

2. The process according to claim 1, wherein the isolating step comprises cooling the reaction mixture and adjusting pH of the reaction mixture to between 7 and 12 to form a solid comprising 2-(2-amino propan-2-yl)-N-(4-fluorobenzyl)-5-hydroxy-1-methyl-6-oxo-1,6-dihydro pyrimidine-4-carboxamide (Formula III).

3. The process according to claim 2, wherein the solid is filtered and washed with water.

4. The process according to claim 2, further comprising the step of purifying 2-(2-amino propan-2-yl)-N-(4-fluorobenzyl)-5-hydroxy-1-methyl-6-oxo-1,6-dihydro pyrimidine-4-carboxamide (Formula III) by washing said solid with an alcohol.

5. A process for preparing Raltegravir or pharmaceutically acceptable salts thereof prepared from 2-(2-amino propan-2-yl)-N-(4-fluorobenzyl)-5-hydroxy-1-methyl-6-oxo-1,6-dihydro pyrimidine-4-carboxamide (Formula III), wherein the process comprises the steps of claim 1.

## Patentansprüche

1. Verfahren zur Herstellung von 2-(2-Amino-propan-2-yl)-N-(4-fluorbenzyl)-5-hydroxy-1-methyl-6-oxo-1,6-dihydropyrimidin-4-carboxamid von Formel III, umfassend:
a. Debenzylieren von Benzyl(2-{4-[(4-fluorbenzyl)carbamoyl]-5-hydroxy-1-methyl-6-oxo-1,6-dihydropyrimidin-2-yl}propan-2-yl)carbamat (Formel IV) in Gegenwart von einer Säure; und
b. Isolieren von 2-(2-Aminopropan-2-yl)-N-(4-fluorbenzyl)-5-hydroxy-1-methyl-6-oxo-1,6-dihydropyrimidin-4-carboxamid (Formel III). 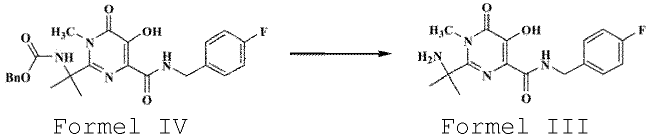 wobei die Säure Bromwasserstoffsäure ist; und wobei das Debenzylieren der Verbindung der Formel IV bei einer Temperatur zwischen 40 °C und 100 °C ausgeführt wird.

2. Verfahren nach Anspruch 1, wobei der Isolierschritt das Kühlen des Reaktionsgemischs und Anpassen des pH des Reaktionsgemischs auf zwischen 7 und 12 umfasst, um einen Feststoff zu bilden, der 2-(2-Aminopropan-2-yl)-N-(4-fluorbenzyl)-5-hydroxy-1-methyl-6-oxo-1,6-dihydropyrimidin-4-carboxamid (Formel III) umfasst.

3. Verfahren nach Anspruch 2, wobei der Feststoff gefiltert und mit Wasser gewaschen wird.

4. Verfahren nach Anspruch 2, ferner umfassend den Schritt des Reinigens von 2-(2-Aminopropan-2-yl)-N-(4-fluorbenzyl)-5-hydroxy-1-methyl-6-oxo-1,6-dihydropyrimidin-4-carboxamid (Formel III) durch Waschen des Feststoffs mit einem Alkohol.

5. Verfahren zum Herstellen von Raltegravir oder pharmazeutisch verträglichen Salzen davon, das bzw. die aus 2-(2-Aminopropan-2-yl)-N-(4-fluorbenzyl)-5-hydroxy-1-methyl-6-oxo-1,6-dihydropyrimidin-4-carboxamid (Formel III) hergestellt wird bzw. werden, wie es durch das Verfahren nach Anspruch 1 erhalten wurde.

## Revendications

1. Procédé de préparation de 2-(2-amino propan-2-yl)-N-(4-fluorobenzyl)-5-hydroxy-1-méthyl-6-oxo-1,6-dihydropyrimidine-4-carboxamide selon la Formule III, comprenant :
a. une débenzylation de benzyl(2-{4-[(4-fluorobenzyl)carbamoyl]-5-hydroxy-1 -méthyl-6-oxo-1,6-dihydropyrimidin-2-yl}propan-2-yl )carbamate (Formule IV) en présence d'un acide ; et
b. l'isolation de 2-(2-amino propan-2-yl)-N-(4-fluorobenzyl)-5-hydroxy-1-méthyl-6-oxo-1,6-dihydropyrimidine-4-carboxamide (Formule III). 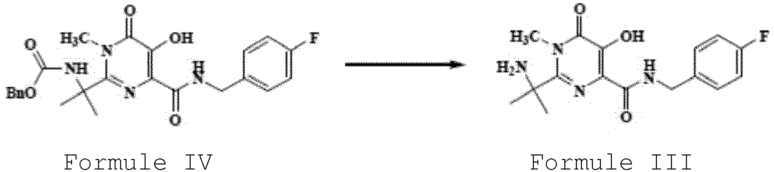 où l'acide est de l'acide bromhydrique ; et où la débenzylation du composé selon la Formule IV est effectuée à une température entre 40 °C et 100 °C.

2. Procédé selon la revendication 1, dans lequel l'étape d'isolation comprend un refroidissement du mélange réactionnel et l'ajustement du pH du mélange réactionnel à une valeur entre 7 et 12 afin de former un solide comprenant le 2-(2-amino propan-2-yl)-N-(4-fluorobenzyl)-5-hydroxy-1-méthyl-6-oxo-1,6-dihydropyrimidine-4-carboxamide (Formule III).

3. Procédé selon la revendication 2, dans lequel le solide est filtré et lavé avec de l'eau.

4. Procédé selon la revendication 2, comprenant en outre l'étape de purification du 2-(2-amino propan-2-yl)-N-(4-fluorobenzyl)-5-hydroxy-1-méthyl-6-oxo-1,6-dihydropyrimidine-4-carboxamide (Formule III) en lavant ledit solide avec un alcool.

5. Procédé de préparation de raltégravir ou de sels pharmaceutiquement acceptables de celui-ci préparés à partir de 2-(2-amino propan-2-yl)-N-(4-fluorobenzyl)-5-hydroxy-1-méthyl-6-oxo-1,6-dihydropyrimidine-4-carboxamide (Formule III) tel qu'obtenu par le procédé selon la revendication 1.
